Europäisches Patentamt

European Patent Office

Office européen des brevets

(19)

(11) Numéro de publication : **0 288 327 B1**

(12) ## FASCICULE DE BREVET EUROPEEN

(45) Date de publication du fascicule du brevet :
**29.04.92 Bulletin 92/18**

(51) Int. Cl.⁵ : **G06F 15/20**

(21) Numéro de dépôt : **88400197.5**

(22) Date de dépôt : **28.01.88**

(54) **Procédé et dispositif de traitement d'un signal numérisé du type comportant des phases à variations lentes, représentatives du phénomène étudié, et des phases à variations rapides.**

(30) Priorité : **28.01.87 FR 8701001**

(43) Date de publication de la demande :
**26.10.88 Bulletin 88/43**

(45) Mention de la délivrance du brevet :
**29.04.92 Bulletin 92/18**

(84) Etats contractants désignés :
**AT BE CH DE ES GB GR IT LI LU NL SE**

(56) Documents cités :
**DE-A- 2 605 131**
**FR-A- 2 232 880**
**GB-A- 2 157 000**
**US-A- 3 624 497**

(56) Documents cités :
**IEEE TRANSACTIONS ON BIOMEDICAL ENGI-NEERING, vol. BME-22, no. 3, mai 1975, pages 196-202, New York, US; J.H.J. ALLUM et al.: "MITNYS-II-A digital program for on-line analysis of nystagmus"**

(73) Titulaire : **INSTITUT NATIONAL DE LA SANTE ET DE LA RECHERCHE MEDICALE (INSERM) 101, rue de Tolbiac F-75654 Paris Cédex 13 (FR)**

(72) Inventeur : **Arzi, Mohammad Impasse des Etourneaux Bât. A3 F-38090 Villefontaine (FR)**

(74) Mandataire : **Bugnon-Hays, Claudine PATCO S.A. 10, rue Vivienne F-75002 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un dispositif pour le traitement de signaux numérisés comportant des phases au cours desquelles cette amplitude varie rapidement, et des phases au cours desquelles cette amplitude dudit signal varie lentement, le signal utile étant représenté par les phases à variations lentes.

Ce dispositif concerne tous types de signaux électriques numérisés, recueillis par un capteur de type connu et, notamment la classification automatique des phases lentes et des phases rapides d'un signal électrique. A titre d'exemple particulier, on citera le traitement de signaux nystagmiques, recueillis par des électrodes périorbitales placées sur les canthis de l'oeil, en vue de l'analyse du réflexe vestibulooculaire.

Les signaux de ce type, et en particulier le signal nystagmique, présentent une alternance de phases rapides, dites saccades, et de phases lentes dites compensatoires.

Pour étudier les phénomènes liés à de tels signaux, il est nécessaire de détecter et d'éliminer les composantes rapides, et dans un deuxième temps de reconstruire le signal cumulé des phases lentes, appelé dans le cas de signaux nystagmiques "position cumulée de la phase lente (PCPL)", en mettant bout-à-bout les phases lentes, et en interpolant pendant la durée des phases rapides.

On a proposé, dans l'art antérieur, différents procédés et/ou dispositifs de traitement automatiques de tels signaux.

Le brevet français FR 2 232 880 a pour objet un dispositif permettant de cumuler les sections à variation utile d'un signal constitué par une succession d'impulsions.

Le brevet allemand 2 605 131 décrit un procédé permettant d'analyser le nystagmus oculaire, et consistant à cumuler les phases lentes du signal et à extrapoler la phase lente à la phase rapide qui suit.

L'article paru dans I.E.E.E. Transaction on Bio-Medical Engineering, Mai 1975, Vol. BME-22 N° 3, pages 196 à 202, décrit un programme numérique pour le traitement en ligne de signaux nystagmiques. Ce programme utilise, pour la reconnaissance de saccades et la construction de la PCPL, une multitude de critères, et notamment la valeur absolue de la vitesse, le changement de la pente du signal de la position de l'oeil, la pente du signal de la position de l'oeil.

Au cours du traitement, les différents critères et seuils sont continuellement mis à jour. L'ordinateur calcule la valeur instantanée de la vitesse de l'oeil, et compare cette valeur à une fonction qui dépend d'une estimation courante de la vitesse de la phase lente. La vitesse de la phase lente de l'oeil est continuellement mise à jour. Trois tests séparés comparent les valeurs instantanées aux valeurs estimées et permettent de détecter les saccades. Une partie de ces comparaisons est basée sur un modèle d'un système oculomoteur considéré comme normal.

Dans un article paru dans Computer Methods and Programs in Biomedicine, 1985, 21, pages 81 à 88, on propose un algorithme pour la détection de saccades utilisant un filtre numérique non récursif et adaptatif. Ce programme calcule les caractéristiques des saccades, et les erreurs causées par lesdites saccades non reconnues sont minimisées en faisant le traitement sur un nombre important de saccades. Un filtre numérique reçoit à l'entrée le signal nystagmique. Sa sortie est utilisée pour détecter une saccade et pour fournir un signal maximal quand l'entrée correspond à une saccade.

Ces procédés présentent une fiabilité insuffisante, en raison de nombreuses hypothèses simplificatrices sur les signaux et systèmes étudiés, l'efficacité des procédés de type connu n'est satisfaisante que pour des signaux offrant un bonne prédectibilité.

La présente invention, comme revendiquée, a pour objet un dispositif assurant, avec un taux d'erreur acceptable quel que soit le type de signal, le traitement automatique d'un signal du type comportant une succession de phases à variations lentes représentatives du phénomène étudié et de phases à variations rapides, en rejetant lesdites phases rapides et en extrapolant les phases à variations lentes. Plus particulièrement, il comporte des moyens permettant d'assurer la réalisation d'au moins les étapes suivantes :
– le signal est échantillonné et digitalisé, la fréquence d'échantillonnage étant supérieure à la fréquence de SHANNON, c'est-à-dire au double de la bande passante du filtre électronique avant échantillonnage ;
– le signal numérisé est dérivé par un filtre numérique dérivateur ;
– une fonction d'appartenance globale de chacune des valeurs échantillonnées à la phase lente est construite au cours d'un premier balayage de la dérivée du signal numérique échantillonné ;
– la forme globale du signal est détectée en faisant un ajustement de la courbe sur la dérivée, avec le critère des moindres carrés pondérés par la fonction d'appartenance globale ;
– une fonction d'appartenance fine à des phases lentes des valeurs échantillonnées, est construite au cours d'un deuxième balayage en se référant à la forme globale du signal ;
– les valeurs échantillonnées sont classées selon leur appartenance aux phases rapides ou aux phases lentes ;
– les phases rapides sont éliminées ;

– le signal cumulé des phases lentes est construit ;

Pour calculer la dérivée du signal en un point, on fait une approximation polynômiale du signal, avec le critère des moindres carrés.

On construit ainsi un tableau numérique représentant la dérivée du signal à traiter.

On définit n bandes horizontales en divisant la dynamique du signal'par une valeur N prédéterminée.

Le dispositif selon l'invention définit deux types de fonction d'appartenance :

**locale** (fine) et **globale** (grossière). Une fonction d'appartenance est locale si la valeur d'appartenance de tous les points est divisée par son maximum local. De la même manière, une fonction d'appartenance est globale si la valeur d'appartenance de tous les points est divisée par son maximum global.

Pour la définition de la fonction d'appartenance, on se base sur la dérivée du signal. La définition est guidée par le sens de la notion de "lent" comparée aux phases rapides. Le contexte (univers du discours) est l'ensemble de tous les points du signal. Ces points sont désignés par Pi (il s'agit des points de la courbe de la dérivée), et leur coordonnées par (ti, y(ti)).

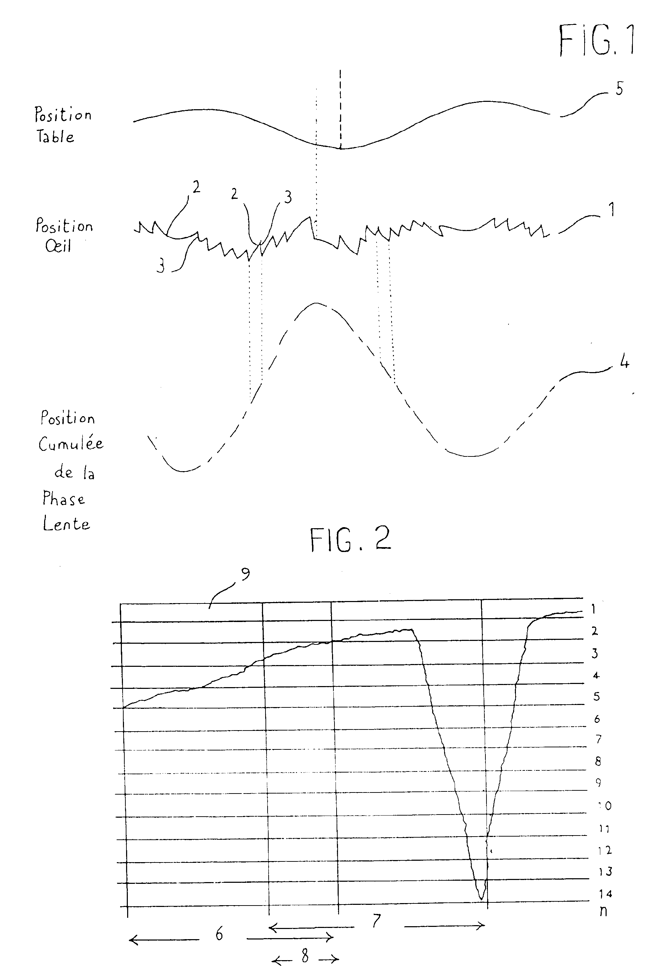

A cette étape, une fonction d'appartenance grossière (globale) pour la notion "lent" est définie. Pour définir le terme "lent", on construit un histogramme qui représente le nombre de points situés dans les bandes de points dont les coordonnées satisfont les relations suivantes (bandes rectilignes voir les figures 1 et 2) :

$$MI + n < y(ti) \leqq MI + (n + 1).e \; ; \; O<n<N \; ; \quad (1)$$

où

MI=MINi(y(ti)) et N s'obtient de la relation suivante :

$$N.e = MAXi(y(ti))-MINi(y(ti)) \; ; \quad (2)$$

Cet histogramme est construit dans une fenêtre temporelle mobile de largeur w. Les deux paramètres : w et e doivent être choisis suivant les informations a priori que nous avons du signal. Plus précisemment, à l'intérieur de cette fenêtre, le signal de la phase lente, qui ressemble au signal de la stimulation, ne doit pas avoir trop de variations. Par exemple, pour les stimulations sinusoïdales, on choisit la largeur w, d'une fraction de la période du signal (préférentiellement, il a été choisi 1/4 pour cette fraction). e détermine la résolution permettant de détecter les petites saccades. Ce paramètre doit être plus petit que la plus petite saccade que l'on veut éliminer. Dans le cas de l'analyse du nystagmus oculaire un choix raisonnable est le suivant :

$$e = (MAXi(y(ti))-MINi(y(ti)))/20 \quad (3)$$

Cette valeur e donne N=20 dans les formules (1) et (2).

Cette manière de définir la notion "lent" est justifiée par le fait que pour les parties lentes du signal, il y aura plus de points dans chaque bande. Mais il est clair que cette information est insuffisante et comme nous allons le voir par la suite, il faut explorer davantage le signal.

L'histogramme est d'abord construit dans deux fenêtres temporelles qui se chevauchent (6,7).

Préférentiellement, la largeur d'intersection des deux fenêtres, correspond au tiers de la largeur de chaque fenêtre (6,7). Pour chaque bande de points dont la valeur des ordonnées est dans l'intervalle : MI+ne, MI+(n+l).e , on obtient deux distributions de nombre de points : ml(n) et m2(n) (m(n) étant le nombre de points dans la bande numéro n) correspondant aux histogrammes construits dans les deux fenêtres temporelles (6,7). Ensuite un troisième histogramme est construit dans l'intersection des deux fenêtres précédentes et une troisième distribution de nombre de points est obtenue pour les mêmes bandes que nous désignerons par m3(n).

Pour chaque point Pi, dont la valeur de l'ordonnée est dans l'intervalle[n e, (n+l).e] , et qui est dans l'intersection des deux fenêtres temporelles (6,7) nous affectons la valeur suivante comme valeur d'appartenance aux phases lentes (voir les figures 1 et 2) :

$$U - lent(Pi) = m1(n). m2(n).m3(n) \quad (4)$$

En terminologie floue, cette dernière équation est équivalente au "produit algébrique" ou "intersection faible" de la fonction d'appartenance de "lent", définie dans trois contextes différents. En d'autres termes, l'appartenance d'un point aux phases lentes est "confirmée" dans une petite fenêtre (8)(intersection des deux fenêtres 6 et 7) par son apppartenance explorée dans deux fenêtres plus larges qui contiennent cette trosième.

Pour avoir :$U -lent (Pi) \in [0,1]$ et pour s'assurer que :

$$MAX (U - lent (Pi)) = 1,$$

le degré d'appartenance de tous les points est divisé par sa valeur maximum. A ce stade, ceci est fait globalement, c'est-à-dire, le maximum est calculé globalement sur une durée assez large qui englobe les points caractéristiques du signal. Par exemple pour une stimulation sinusoïdale, la durée est au moins la demi période du signal. En faisant ceci, on s'assure des points sûrs des phases lentes du nystagmus, influençant fortement la définition de la fonction d'appartenance des phases lentes.

Pour d'autres types de stimulations, il suffit que cette durée englobe une partie du signal qui contient assez de variations temporelles de la phase lente et de nombreuses saccades (par exemple une quinzaine). Ceci nous donne ce que nous appellerons : **"une fonction d'appartenance globale"**. Après l'exploration de la fonction d'appartenance globale, la forme globale de la courbe de vitesse lente est révélée en faisant un meilleur ajustement de courbe par le critères des "moindres carrés flous". Ceci est réalisé en minimisant la somme suivante

$$F = \varepsilon i \; U \text{-lent (Pi).(y(ti)-f(ti))}^2 \quad (5)$$

dans cette formule, U - lent (Pi) est le degré d'appartenance de chaque point défini plus haut, et f(ti) est la fonction qui minimise la somme (5). Cette dernière fonction servira de référence pour les raffinements ultérieurs de la fonction d'appartenance. Il suffit qu'elle soit la plus proche possible de la forme de la-courbe de la vitesse des phases lentes, mais il n'est pas nécessaire qu'elle soit exactement la même courbe que la dernière. Une méthode pour déterminer cette fonction, est de la restreindre à une classe finie de fonctions qui semble appropriée (par exemple les polynômes de degré fini). Ensuite, en utilisant une méthode itérative, trouver la fonction qui donne la plus petite somme de carrés des erreurs pondérées par la fonction d'appartenance (à chaque itération, il faut minimiser F).

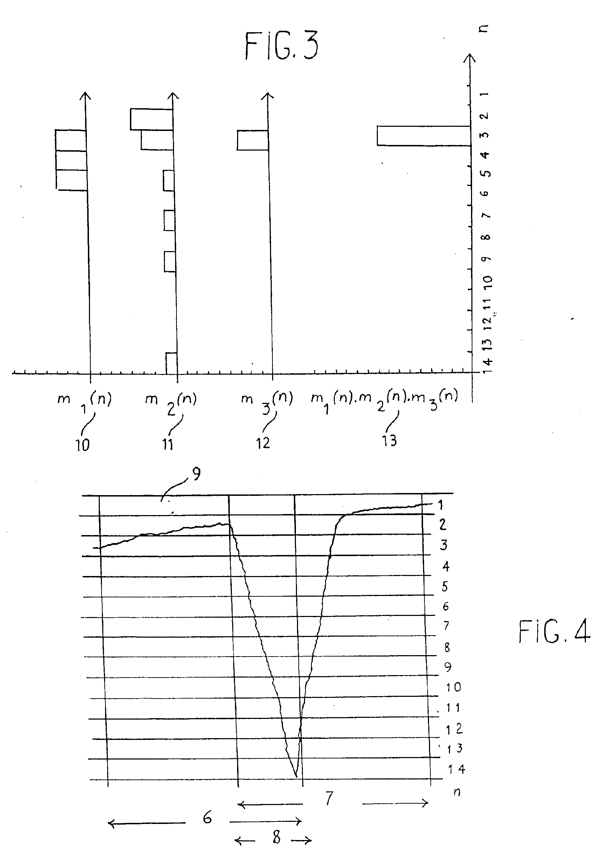

D'une manière similaire à la première étape, on explore alors une deuxième fois le signal pour redéfinir plus finement la fonction d'appartenance en faisant référence à la forme globale de la vitesse des phases lente obtenue en deuxième étape. Cette fois les histogrammes sont construits pour les bandes de points dont les ordonnées satisfont la relation suivante (bandes curvilignes voir la figure 4) :

$$n.e < (y(ti)\text{-}f(ti)) \leq (n + 1).e \; ; \; M1 \leq n < M2 \; ; \quad (7)$$

où M1 et M2 s'obtiennent des relations suivantes :

$$M1.e = MINi \; (y(ti)\text{-}f(ti)) \quad (8)$$
$$M2.e = MAXi \; (y(ti)\text{-}f(ti)) \quad (9)$$

et où e est défini comme en première étape. f(ti) est la fonction obtenue en deuxième étape.

Comme en première étape, nous allons obtenir trois distributions de nombre de points : m1(n), m2(n), m3(n) pour les bandes définies ci-dessus. La valeur d'appartenance de chaque point est définie par la relation suivante :

$$U \text{-} lent \; (Pi) = ((M3\text{-}|n|)/M3). \; m1(n).m2(n).m3(n) \quad (10)$$

où est défini dans (7) et :
M3 = MAX($|M1|$, $|M2|$) ; M1 et M2 sont données par les relations (8) et (9).

Le dispositif selon la présente invention est notamment destiné au traitement du signal nystagmique ou au traitement d'images. Dans le cas du traitement d'images, le signal traité est délivré par une caméra du type C.C.D.

Il comporte au moins une mémoire de stockage du signal numérique, une mémoire de stockage pour le stockage de la dérivée dudit signal numérique, une mémoire de stockage de la fonction d'appartenance globale, une mémoire de stockage du signal cumulé des phases lentes, et un processeur arithmétique gérant les différentes étapes dudit traitement.

D'autres avantages et modes de réalisation ressortiront mieux de la description qui va suivre, et qui s'appuie sur les figures où :

– La figure 1 représente un exemple de signal nystagmique.
– La figure 2 représente une série de trois fenêtres temporelles utilisées pour la construction de la fonction d'appartenance globale.
– La figure 3 représente les histogrammes obtenus dans le cas d'une phase lente.
– La figure 4 représente une série de trois fenêtres temporelles pour les cas où l'intersection des deux fenêtres est une phase rapide.

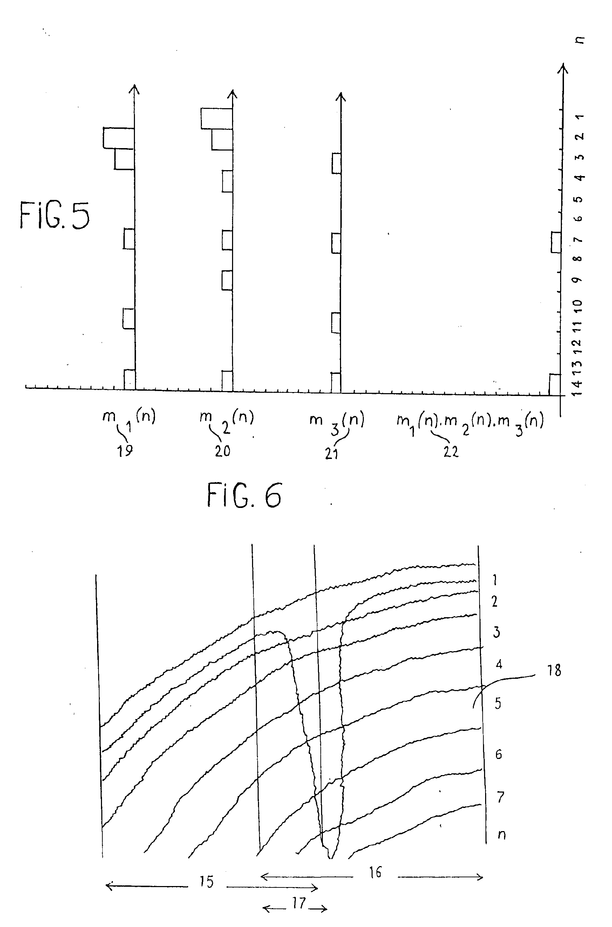

– La figure 5 représente les histogrammes obtenus dans le cas d'une phase rapide.
– La figure 6 représente une série de trois fenêtres à bande curviligne pour la détermination de la fonction d'appartenance fine.

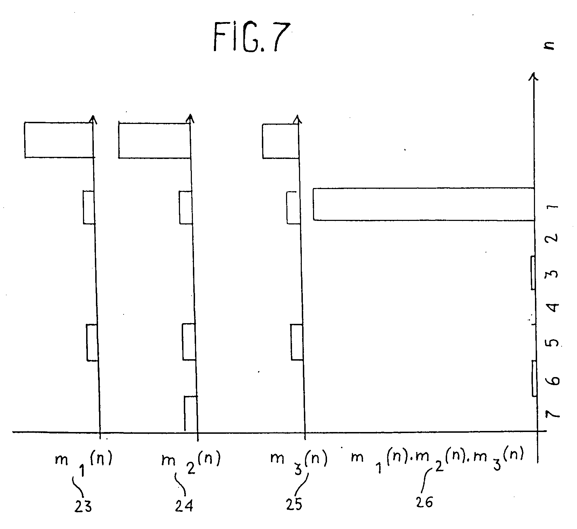

– La figure 7 représente les histogrammes obtenus au cours de la phase de détermination de la fonction d'appartenance fine.

La présente description concerne plus particulièrement le traitement du signal nystagmique, sans pour autant être limitatif de ce type de signal.

Les signaux nystagmiques sont obtenus en recueillant les potentiels électrooculomoteurs à l'aide, par exemple, de capteurs périorbitaux disposés sur les canthis externes de l'oeil d'un sujet placé au centre d'un plateau tournant autour d'un axe. Le réfexe vestibulooculaire impose un mouvement compensatoire de l'oeil ayant une vitesse similaire mais de sens opposé à celui de la tête. A partir d'une certaine amplitude de déplacement de la tête, on constate un retour rapide de l'oeil vers sa position initiale. Ce retour rapide est appelé saccade et correspond à la phase rapide du signal. La phase lente correspond à la phase compensatoire.

Il faut donc traiter le signal délivré par les capteurs périorbitaux afin d'éliminer les phases rapides et de restituer un signal correspondant à la position cumulée des phases lentes.

La courbe de la position de l'oeil (1) sur la figure 1, comporte des phases lentes (2) et des phases rapides (3).

La courbe des positions cumulées de la phase lente (4) est construite en supprimant les phases rapides (3) et en mettant bout-à-bout les phases lentes (2) et en faisant une interpolation pendant lesdites phases rapides (3). Il est alors possible de comparer la courbe représentant la position cumulée de la phase lente (4) à la courbe représentant le mouvement de l'assimilation (5).

Pour les signaux nystagmiques, ainsi que pour tous les signaux présentant des phases lentes et des phases rapides, les concepts "lent" et "rapide" sont relatifs. Il n'existe pas de seuil absolu en-deçà duquel une séquence peut de façon certaine être attribuée à une phase lente, et au-delà duquel la séquence peut être attribuée de façon certaine à une phase rapide.

La première étape consiste à l'acquisition du signal et à sa digitalisation.

Le signal recueilli par les électrodes périorbitales est amplifié, filtré de façon à éliminer le bruit aléatoire ou prédictible, par exemple les perturbations provenant du secteur, puis digitalisé à l'aide d'un convertisseur analogique digital.

La fréquence d'échantillonnage du signal est au moins égale à la fréquence de SHANNON correspondant au double de la bande passante du filtre électronique utilisé avant échantillonnage.

Le signal prétraité est numérisé et ensuite stocké dans la mémoire d'un ordinateur.

La deuxième étape consiste à dériver le signal point par point.

On calcule la dérivée en faisant une approximation polynômiale du signal, avec le critère des moindres carrés.

Pour calculer la dérivée du signal au point ti, on considère les deux k + 1 points : ti - k, ti - k + 1, ..., ti + k.

On réalise ensuite un changement de variable de la forme

n = (tj - ti) / dt ; j = i - k, i - k + 1, ..., i + k, dt étant l'intervalle d'échantillonnage.

Pour une approximation polynômiale du deuxième degré, on obtient la formule suivante :

$$f(n) = a0 + a1 \times n + a2 \times n^2$$

Pour calculer les paramètres a0, a1, a2, on minimise la somme suivante :

$$F = \Sigma_j^k = -k \, (f(j) - y(j))^2,$$

y (j) étant les ordonnées des points acquis.

On aboutit aux équations suivantes :

$$(2.k + 1).a0 + (\Sigma_j^k = -kj).a1 + (\Sigma_j^k = -k\,j^2).a2 = \Sigma_j^k = -k\,y(j)$$

$$(\Sigma_j^k = -k\,j).a0 + (\Sigma_j^k = -k\,j^2).a1 + (\Sigma_j^k = -k\,j^3).a2 = \Sigma_j^k = -kj.y(j)$$

$$(\Sigma_j^k = -k\,j^2).a0 + (\Sigma_j^k = -k\,j^3).a1 + (\Sigma_j^k = -kj^4).a2 = \Sigma_j^k = -kj^2.y(j)$$

les sommes sur les puissances impaires de j étant nulles, on obtient les équations suivantes :

$$(2.k + 1).a0 + (\Sigma_j^k = -kj^2).a2 = \Sigma_j^k = -ky(j)$$

$$(\Sigma_j^k = -k\,j^2).a1 = \Sigma_j^k = -kj.y(j)$$

$$(\Sigma_j^k = -k\,j^2).a0 + (\Sigma_j^k = -kj^4).a2 = \Sigma_j^k = -kj^2.y(j)$$

d'où on peut calculer facilement les paramètres a0, a1 et a2.

D'autre part, en dérivant la formule de la parabole, on a :

df(n)/dn=a1+2.a2.n ; n=-k, -k+1, ..., +k ; pour n=0, on obtient :

$$df(n)/dn = a1 = (\Sigma_j^k = -k\,j.y(j)\,)/(\Sigma_j^k = -k\,j^2)$$

Il ne reste qu'à refaire un changement inverse de variable pour obtenir la formule de la dérivée :

n=(tj-ti)/Δt ===> tj=ti+Δt.n ===> df/dtj=(df/dn)/Δt

D'autre part, nous avons :

y(tj)=y(ti+n.Δt) ; n=-k,-k+1, ..., +k

Nous obtiendrons finalement la formulation exacte pour une dérivation parabolique comme suit :

$$dy(ti)/dt = (1/\Delta t).[(\Sigma_j^k = -kj.y(ti + j.\Delta t)/(\Sigma_j^k = -k\,j^2)]$$

La routine de dérivation accepte comme l'un es paramètres d'appel, le nombre de points (2*K+1). Pour K=2, la valeur de la dérivée du signal au point ti est :

$$(1/(10.\Delta t)).[-2.y(ti-2.\Delta t)-y(ti-\Delta t) + y(ti + \Delta t) + 2.y(ti + 2.\Delta t)]$$

On construit ensuite sur la dérivée du signal ainsi calculée un réseau de bandes (9) rectilignes divisant la dynamique du signal par un nombre N prédéterminé.

On choisira une valeur N sensiblement égale à 20. Ce paramètre détermine la résolution de l'algorithme pour détecter les petites saccades. Ce paramètre doit être plus petit que la plus petite saccade que l'on veut éliminer.

La troisième étape consiste à déterminer une fonction d'appartenance globale de chacune des valeurs échantillonnées. La fonction d'appartenance globale à la phase lente est construite au cours d'un premier balayage de la dérivée du signal numérique stocké en mémoire.

Pour construire la fonction d'appartenance globale, on déplace une série de trois fenêtres temporelles sur la dérivée du signal mémorisé.

Lesdites fenêtres temporelles (6), (7), (8), représentées sur les figures 2 et 4, ont une largeur temporelle telle que, à l'intérieur desdites fenêtres, le signal de la phase lente ne présente pas trop de variations. Cette largeur est déterminée à priori en fonction des caractéristiques du signal. Pour un signal utile à priori sensiblement sinusoïdal, une largeur temporelle des fenêtres de l'ordre du quart de la période dudit signal convient bien. La fenêtre (8) est constituée par l'intersection de la fenêtre (6) et de la fenêtre (7). La largeur de la fenêtre d'intersection (8) est égale au tiers de la largeur des fenêtres (6) ou (7).

L'étape suivante consiste à compter le nombre de points du signal numérisé et mémorisé présent dans chacune des bandes à l'intérieur des fenêtres (6), (7) et (8), et à réaliser l'histogramme des distributions du nombre de points dans lesdites bandes (9). On obtient ainsi trois histogrammes (10), (11) et (12) correspondant à la distribution des points dans les N bandes (9) des fenêtres respectivement (6), (7) et (8). Les histogrammes (10), (11), (12), représentent en ordonnées le nombre de points du signal dont l'amplitude est comprise dans la bande représentée en abscisses.

A partir de ces trois histogrammes, on construit un histogramme produit (13) par multiplication pour chacune des bandes représentées en abscisses du nombre de points représentés en ordonnées dans chacun des histogrammes (10), (11) et (12).

Pour chacun des points d'une bande (9) de la fenêtre d'intersection (8), on affecte une valeur d'appartenance aux phases lentes définie par la valeur en ordonnées de l'histogramme (13), correspondant à la bande (9) contenant ledit point.

L'appartenance d'un point aux phases lentes est confirmée dans une petite fenêtre (8) correspondant à l'intersection des deux fenêtres (6) et (7), par son appartenance explorée dans les deux fenêtres plus larges (6) et (7).

La figure 4 représente un exemple de balayage d'une phase rapide. Les histogrammes résultants (10), (11), (12), conduisent à la construction d'un histogramme des valeurs d'appartenance aux phases lentes des différents points présents dans les bandes (9) de la fenêtre (8) comportant des valeurs d'appartenance beaucoup plus faibles.

Les fenêtres temporelles (6), (7), (8) sont ensuite déplacées d'un pas correspondant à la largeur de la fenêtre d'intersection (8).

Lorsque l'ensemble l'ensemble du signal numérisé et mémorisé a été balayé, on normalise les valeurs d'appartenance aux phases lentes en divisant chaque valeur d'appartenance attribuée à chacun des points du signal numérisé et mémorisé par la valeure maximale de la valeur d'appartenance obtenue au cours du balayage.

Les valeurs d'appartenance normalisées sont comprises entre 0 et 1.

L'étape suivante consiste à déterminer la forme globale de la phase lente. Pour cela, on déterminera la fonction f (ti) qui minimisera selon le critère des moindres carrés la somme suivante :

$$F = \varepsilon i \, U\_lent \, (pi) \times (y \, (ti) - f \, (ti))^2$$

où U_lent (pi) correspond au degré d'appartenance attribué à chaque point du signal numérisé, y (ti) correspondant à la valeur du signal mémorisé à l'instant ti, et la fonction f (ti) est la fonction qui minimise cette somme. Cette dernière fonction servira de référence pour la construction plus précise du signal ultérieurement.

Lorsqu'on ne possède aucune information sur la forme du signal utile, on l'assimilera localement à une fonction polynômiale dont on déterminera les paramètres.

Par une méthode itérative, on trouvera la fonction, et donc les paramètres, qui donne la plus petite somme de carrés des erreurs pondérés par la fonctions d'appartenance.

Notamment, lorsque la forme globale des phases lentes est du type sinusoïdal, on utilisera pour la fonction f (ti):

$$A \text{ sinus } (c \, ti) + N \text{ cosinus } (C \, ti).$$

Si on possède des informations à priori sur le signal utile, on pourra améliorer la précision de l'approximation. A et B sont les deux paramètres à calculer en minimisant la somme F, et C est la fréquence prédictible du signal correspondant aux phases lentes.

L'étape suivante consiste à construire la fonction d'appartenance fine à la phase lente des valeurs échantillonnées. Cette fonction d'appartenance fine est construite au cours d'un deuxième balayage du signal par trois fenêtres (15), (16), (17) représentées en figure (6) La fenêtre (17) est constituée par l'intersection des fenêtres (15) et (16).

Comme pour la construction de la fonction d'appartenance globale, on construit trois histoyrammes, (23), (24), (25) correspondant respectivement aux fenêtres (15), (16), (17), ainsi qu'un histogramme produit (26). Les histogrammes (23), (24), (25) représentent le nombre de points par bande curviligne (18), dont les ordonnées satisfont à l'intérieur des fenêtres (15), (16), (17), la relation suivante :

$n \times e < (y \, (ti) - f \, (ti)) \leqq (n + 1) \times e$, à l'intérieur des fenêtres (15), (16), (17) ; $M1 \leqq n \leqq M2$

où M1 et M2 s'obtiennent des relations suivantes :

$$M1 \times e = MIN\ i\ (y\ (ti) - f\ (ti))$$
$$M2 \times e = MAX\ i\ (y\ (ti) - f\ (ti))$$

où e correspond à la largeur d'une bande (18) et f (ti) correspond à la fonction obtenue lors de la détermination de la forme globale. Ces histogrammes sont construits à l'intérieur des fenêtres (15), (16), (17).

On obtient ainsi trois distributions de nombre de points : m1 (n), m2 (n), m3 (n) pour les bandes définies ainsi.

La valeur d'appartenance de chaque point est définie par la relation suivante :

$$U\_lent\ (pi) = ((M3 - |n|)\ /\ M3) \times m1\ (n) \times m2\ (n) \times m3\ (n)$$

où n est défini par la relation : $M1 \leqq n \leqq M2$ et $M3 = MAX\ (|M1|, |M2|)$,
$K = ((M3 - |n|)\ /\ M3)$ est un poids multiplicatif qui relie la fonction d'appartenance à la distance de chaque joint à f (t).

Pour les points proches de f (t), n = 0 et K = 1et pour les points les plus éloignés de f (t), n = M3 - 1 et K = 1 / M3.

La fonction d'appartenance est cette fois normalisée localement, en divisant la valeur d'appartenance de tous les points par son maximum local. Ce maximum est calculé dans un domaine limité, à l'intérieur de l'intersection des deux fenêtres temporelles (16) et (17) que nous avons définies précédemment.

L'étape suivante a pour objet la construction du signal cumulé des phases lentes du signal.

Chaque point du signal numérisé et mémorisé est classé en fonction de son appartenance aux phases lentes ou rapides.

On considère comme appartenant aux phases lentes tous les points dont le degré d'appartenance dans sa fonction d'appartenance locale est suffisamment élevé. Le choix de la valeur de la coupure détermine la plus petite saccade que l'on veut éliminer. Un choix raisonnable pour les signaux nystagmiques est de 0,9. Le signal traité sera d'autant plus lisse que cette valeur de coupure sera élevée.

Tous les points dont le degré d'appartenance affecté est inférieur à la valeur de coupure sont éliminés et remplacés par une interpolation notamment polynômiale entre les phases lentes. Une interpolation linéaire donne des résultats satisfaisants. Pour cette interpolation linéaire, on choisit comme pente la valeur de la fin de la phase lente avant la phase rapide éliminée. Si l'appartenance des points à la fin de la phase lente n'est pas suffisamment élevée, on choisit la pente de la vitesse de la partie de cette phase qui précède la saccade et qui a la valeur d'appartenance la plus élevée. Le signal numérique ainsi traité est ensuite stocké dans un fichier permettant son exploitation.

La présente invention est avantageusement mise en oeuvre à l'aide d'un ordinateur comportant une mémoire vive, une carte d'horloge pour la temporisation, une carte d'entrée-sortie analogique/numérique, une carte d'entrée-sortie numérique, des unités de stockage en mémoire et de visualisation. Le dispositif selon la présente invention comporte également des moyens d'acquisition du signal sous forme analogique. La sortie peut êre constituée par un écran de visualisation du signal après traitement ou par tout autre périphérique adapté au type de signaux traités.

La présente invention ne se limite pas aux exemples décrits, mais s'étend à toutes ces variantes appliquées au traitement de tous types de signaux.

## Revendications

1. Dispositif pour le traitement automatique d'un signal numérisé du type comprenant une succession de phases à variations lentes représentatives du phénomène étudié, et de phases à variations rapides, consistant à rejeter lesdites phases rapides et à extrapoler les phases à variations lentes, ledit signal étant échantillonné et digitalisé avec une fréquence d'échantillonnage supérieure à la fréquence de Shannon, le signal numérisé étant dérivé par un filtre numérique dérivateur, caractérisé en ce qu'il comporte des moyens susceptibles

– de construire, au cours d'un premier balayage de la dérivée du signal numérique échantillonné, une fonction d'appartenance globale de chacune des valeurs échantillonnées à la phase lente, cette étape consistant à :

. déplacer une série de trois fenêtres (6,7,8) temporelles sur la dérivée du signal mémorisé, l'une (8) des trois fenêtres temporelles correspondant à l'intersection des deux autres (6,7),

. à compter le nombre de points m1 (n), m2 (n) et m3 (n) du signal numérisé et mémorisé présents dans chacune des bandes rectilignes (9) de la courbe de la dérivée du signal,

. à construire trois histogrammes (10,11,12) correspondant à la distribution des points dans les bandes (9) des fenêtres temporelles (6,7,8),

. à construire un histogramme produit (13) obtenu par multiplication, pour chacune des bandes (9), des nombres de points m1(n), m2(n) et m3(n) représentés en ordonnées dans chacun des histogrammes (10,11,12),

. à effectuer pour chaque point Pi de la courbe de la dérivée une valeur

$$\text{U - lent (Pi)} = \text{m1(n).m2(n).m3(n)}$$

comme valeur d'appartenance aux phases lentes;

– de détecter la forme globale du signal en faisant un ajustement de la courbe sur la dérivée du signal avec le critère des moindres carrés, en minimisant la somme

$$F = \varepsilon i \text{ U - lent (Pi).}(Y(ti)\text{-}f(ti))^2$$

f(ti) étant la fonction qui minimise la somme F;

– de construire, au cours d'un deuxième balayage une fonction d'appartenance fine des valeurs échantillonnées à la phase lente, cette étape consistant à :

. déplacer une série de trois fenêtres (15,16,17) temporelles sur la dérivée du signal mémorisé, l'une (17) des trois fenêtres temporelles correspondant à l'intersection des deux autres (15,16)

. à compter le nombre de points m1(n), m2(n) et m3(n) du signal numérisé et mémorisé présent dans chacune des bandes curvilignes (18) de points dont les ordonnées répondent à la relation suivante :

$$\text{n.e} < (y(ti)\text{-}f(ti)) < (n + 1)e$$

n étant compris entre M1 et M2 ou égal à M1 ou M2, M1 et M2 s'obtenant des relations suivantes :

$$\text{M1.e} = \text{MINi }(y(ti)\text{-}f(ti))$$
$$\text{M2.e} = \text{MAXi}(y(ti)\text{-}f(ti))$$

e correspondant à la largeur d'une bande curviligne (18) et f(ti) correspondant à la fonction obtenue lors de la détermination de la forme globale

. à construire trois histogrammes (19,20,21) correspondant à la distribution des points dans les bandes curvilignes (18) des fenêtres temporelles (15,16,17)

. à construire un histogramme produit (22) obtenu par multiplication, pour chacune des bandes représentées en abscisses, des nombres de points m1(n), m2(n) et m3(n) représentés en ordonnées dans chacun des histogrammes (19,20,21)

. à effectuer pour chaque point Pi de la courbe de la dérivée une valeur

$$\text{U - lent (Pi)} = ((M3\text{-}|n|)/M3) \times m1(n) \times m2(n) \times m3(n)$$

n étant compris entre M1 et M2 ou égal à M1 ou M2 et M3 = MAX $(|M1|, |M2|)$

– de classer selon leur appartenance aux phases lentes ou aux phases rapides, les valeurs échantillonnées,

– d'éliminer les phases rapides,

– de construire un signal cumulé des phases lentes.

2. Dispositif selon la revendication 1, caractériséen ce que la largeur desdites fenêtres temporelles (6), (7), (8), (15), (16), (17), est inférieure au quart de la fréquence de coupure des phases lentes.

3. Dispositif selon la revendication 2, caractérisé en ce que l'intersection des deux fenêtres temporelles (6), (7), constitue une troisième fenêtre temporelle (8), d'une largeur égale au tiers des deux premières fenêtres.

4. Dispositif selon l'une quelconque des revendications 1 à 3, caractérisé en ce que l'excursion du signal dérivé est divisée en bandes d'égale longueur.

5. Dispositif selon la revendication 1, caractérisé en ce que la construction du signal dérivé est réalisée à partir d'une approximation polynômiale du signal avec le critère des moindres carrés.

6. Dispositif selon la revendication 1, caractérisé en ce que la forme globale de la phase lente f(ti) est obtenue en minimisant la somme F = $\varepsilon_i$ 1 (pi) x (y (ti) - f(ti))$^2$, 1 (pi) étant la fonction d'appartenance d'un point de la courbe représentative du signal et y (ti) la valeur du signal à l'instant (ti).

7. Dispositif selon la revendication 2, caractérisé en ce que la forme globale de la phase lente est déterminée en minimisant la somme F = $\varepsilon_i$ 1(pi) x (y (ti) - f(ti))2, f(ti) étant une fonction polynômiale.

8. Dispositif selon l'une quelconque des revendications 1 à 7 caractérisé en ce qu'il est destiné au traitement du signal nystagmique.

9. Dispositif selon l'une quelconque des revendications 1 à 7 caractérisé en ce qu'il est destiné à la classification automatique en phases lentes et en phases rapides d'un signal électrique.

10. Dispositif selon l'une quelconque des revendications 1 à 9, caractérisé en ce qu'il comporte au moins une mémoire de stockage du signal numérique, une mémoire de stockage pour le stockage de la dérivée dudit signal numérique, une mémoire de stockage du signal cumulé des phases lentes et un processeur arithmétique.

## Claims

1. A device for the automatic processing of a digitized signal of the type comprising a sequence of phases with slow variations representative of the studied phenomenon, and of phases with quick variations, consisting in excluding said quick phases and in extrapolating phases with slow variations, said signal being sampled and digitized with a sampling frequency which is above the Shannon frequency, the digitized signal being derived by means of a derivative digital filter, characterized in that it comprises means which are capable of:

– making, during a first scanning of the sampled digital signal derivative, a global membership function for each sampled value of the slow phase, that step consisting in:

. moving a series of three time-division windows (6,7,8) onto the stored signal derivative , one (8) of the three time-division windows corresponding to the intersection of the two others (6,7),

. counting the number of points m1 (n), m2 (n) and m3 (n) of the digitized and stored signal present in each rectilinear band (9) of the signal derivative curve,

. making three histograms (10,11,12) which correspond to the distribution of the points in the rectilinear bands (9) of the time-division windows (6,7,8),

. making one product histogram (13) obtained by multiplying , for each band (9), the number of points m1(n), m2(n) and m3(n) represented as ordinates in each histogram (10,11,12),

. making for each point Pi of the curve of the derivative a value:

$$U - slow\ (Pi)\ =\ m1(n).m2(n).m3(n)$$

as membership value of slow phases;

detecting the global shape of the signal by adjusting the curve on the derivative of the signal with the least error squares criterion, minimizing the sum:

$$F\ =\ \varepsilon i\ U - slow\ (Pi).(Y(ti)-f(ti))^2$$

f(ti) being the function which minimizes the sum F;

– making, during a second scanning, a slight membership function of the sampled values of the slow phase, that step consisting in:

. moving a series of three time-division windows (15,16,17) onto the stored signal derivative , one (17) of the three time-division windows corresponding to the intersection of the two others (15,16),

. counting the number of points m1 (n), m2 (n) and m3 (n)of the digitized and stored signal present in each curved bands (18) of the points, the ordinates of which meet the following relation:

$$n.e<(y(ti)-f(ti)<(n + 1)e$$

n being included between M1 and M2 or equal to M1 or M2, M1 and M2 being obtained from the following relations :

$$M1.e\ =\ MINi\ (y(ti)-f(ti))$$
$$M2.e\ =\ MAXi\ (y(ti)-f(ti))$$

e corresponding to the width of a curved band (18) and f(ti) corresponding to the function obtained when defining the global shape

. making three histograms (19,20,21) which correspond to the distribution of the points in the curved bands (18) of time-division windows (15,16,17)

. making a product histogram (22) obtained by multiplying, for each of the tapes represented in absciss, the number of points m1(n), m2(n) and m3(n) represented in ordinate in each histogram (19,20,21)

. making for each point Pi of the curve of the derivative a value

$$U - slow\ (Pi)\ =\ ((M3-|n|)/M3) \times m1(n) \times m2(n) \times m3(n)$$

n being included between m1 and m2 or equal to m1 or m2 and

$$M3\ =\ MAX\ (|M1|,|M2|)$$

– classifying the sampled values according to their membership of slow phases or quick phases

– eliminating the quick phases,

– making a cumulative signal of the slow phases.

2. A device according to claim 1, wherein the width of said time-division windows (6), (7), (8), (15), (16), (17), is smaller than the fourth of the cut-off frequency of the slow phases.

3. A device according to claim 2, wherein the intersection of both time-division windows (6) and (7) makes a third time-division window (8), of a width equal to the third of the first two windows.

4. A device according to any one of claim 1 to 3, wherein the excursion of the derived signal is divided into bands of equal length.

5. A device according to claim 1, wherein, the making of the derived signal is realized from an polynomial approximation of the signal with the least error squares criterion.

6. A device according to claim 1, wherein the global shape of the slow phase f(ti) is obtained by minimizing the sum $F = \varepsilon_i\ 1\ (pi) \times (y\ (ti) - f(ti))^2$, 1 (pi) being the membership function of a point of the curve representing the signal, and y (ti) the value of the signal at the instant (ti).

7. A device according to claim 2, wherein the global shape of the slow phase is defined by minimizing the sum $F = \varepsilon_i\ 1\ (pi) \times (y\ (ti) - f\ (ti)\ 2$, f (ti) being a polynomial function.

8. A device according to any one of claims 1 to 7, wherein it is meant for the process of nystagmic signal.

9. A device according to any one of claims 1 to 7, wherein it is meant for an automatic classification into slow phases and quick phases of an electric signal.

10. A device according to any one of claims 1 to 9, wherein it has at least one storing memory of the digital signal, a storing memory for storing the derivative of said digital signal, a storing memory of the cumulative signal of slow phases and an arithmetic processor.

**Patentansprüche**

1. Vorrichtung für die automatiche Verarbeitung eines digitalisierten Signals, das aus einer Reihenfolge von Phasen mit langsamen Änderungen, die für das untersuchte Phänomen charakteristish sind, sowie aus Phasen mit schnellen Änderungen besteht, wobei diese die sogenannten schnellen Phasen zurückweist und die Phasen mit langsamen Änderungen extrapoliert; hierbei wird das genannte Signal mit einer im Vergleich zur Shannon-Frequenz höher liegenden Abtastfrequenz abgetastet und digitalisiert, wobei das digitalisierte Signal durch einen ableitenden Digitalfiler abgeleitet wird. Mit dieser Vorrichtung kann :

– im Laufe einer erten Abtastung des Differentials des abgetasteten digitalen Signals eine globale Zugehörigkeitsfunktion zur langsamen Phase für die einzelnen Abtastwerte aufgestellt werden. Diese Etape besteht im

. Verschieben von drei Zeitfenstern (6,7,8) auf dem Differential des gespeicherten Signals, wobei eines der drei Fenster (8) dem Schnittpunkt der beiden anderen (6,7) entspricht,

. Zählen der Anzahl der Punkte m1(n), m2 (n) und m3 (n) des digitalisierten und gespeicherten Signals, die sich auf den einzelnen geradlinigen Bändern (9) der Kurve des Signaldifferentials befinden;

. Aufstellen von drei Histrogrammen (10,11,12), die der Verteilung der Punkte auf den Bändern (9) der Zeitfenster (6,7,8) entsprechen,

. Aufstellen eines Produkt-Histogrammes (13), das für jedes einzelne Band (9) durch Multiplizieren der auf den Ordinaten der einzelnen Histogramme (10,11,12) aufgetragenen Punkte m1(n), m2(n) und m3(n) ermittelt wird,

. Ermitteln eines Wertes U für jeden einzelnen Punkt Pi der Kurve des Differentials

$$U \text{ -langsam (Pi)} = m1(n) * m2(n) * m3(n)$$

Dieser Wert entspricht einem Zugehörigkeitswert für die langsamen Phasen;

Mit dieser Vorrichtung kann :

– die Globalform des Signals ermittelt werden, indem die Kurve auf das Differential des Signals mittels des Kriteriums der kleinsten Fehlerquadrate angepaßt wird, wobei die Summe minimiert wird :

$$F = \Sigma iU \text{ - langsam (Pi)} * (Y(ti) - f(ti))^2$$

wobei f(ti) die Funktion ist, die die Summe F minimiert;

– im Laufe einer zweiten Abtastung eine genaue Zugehörigkeitsfunktion zur langsamen Phase für die Abtastwerte aufgestellt werde. Diese Etape besteht im :

. Verchieben von drei Zeitfenstern (15,16,17) aud dem Differential des gespeicherten Signals, wobei eines der drei Fenster (17) dem Schnittpunkt der beiden anderen (15,16) entspricht,

. Zählen der Anzahl der Punkte m1(n), m2 (n) und m3 (n) des digitalisierten und gespeicherten Signals, die sich auf den einzelnen gekrümmten Punkte-Bändern (18) befinden, deren Ordinaten folgender Gleichung unterliegen :

$$n*e < (y(ti)-f(ti) < (n + 1)e$$

n liegt zwischen M1 und M2 oder ist gleich M1 bzw. M2, M1 und M2 erhält man anhand folgender Gleichungen :

$$M1*e = MINI (y(ti)-f(ti))$$
$$M2*e = MAXI(y(ti)-f(ti))$$

e entspricht der Breite eines gekrümmten Bandes (18) und f(ti) der bei der Bestimmung der Globalform ermittelten Funktion

. Erstellen von drei Histogrammen (19,20,21), die der Verteilung der Punkte auf den gekrümmten Bändern (18) der Zeitfenster (15,16,17) entsprechen,

. Erstellen eines Produkt-Histogrammes (22), das für jedes auf der Abzisse dargestllte einzelne Band (9) durch Multiplizieren der auf den Ordinaten der einzelnen Histogramme (19,20,21)

. Ermitteln eines Wertes U für jeden einzelnen Punkt Pi der Kurve des Differentials

$$U \text{ -langsam (Pi)} = ((M3 -(n)/ M3) * ml (n) * m2(n) * m3(n)$$

n liegt zwischen M1 und M2 oder ist gleich M1 bzw. M2 und für M3 gilt :M3 = MAX ((M1), (M2))

Mit dieser Vorrichtung können (kann) :

– die Abtastwerte nach iher Zugehörigkeit zu den langsamen bzw. schnellen Phasen eingeteilt werden,

– die schnellen Phasen eliminiert werden,

– ein aus den langsamen Phasen kumuliertes Signal erstellt werden.

2. Vorrichtung gemäß Patentanspruch 1, wobei die Breite der Zeitfenster (6), (7), (8), (15), (16) und (17) weniger als 25% der Grenzfrequenz der langsamen Phasen beträgt.

3. Vorrichtung gemäß Patentanspruch 2, wobei der Schnittpunkt der beiden Zeitfenster (6) und (7) ein drittes Zeitfenster (8) darstellt, dessen Breite ein Drittel der Breite der ersten beiden Fenster beträgt.

4. Vorrichtung gemäß der drei Patentansprüche 1 bis 3, wobei die Frequenzabweichung des abgeleiteten Signals in Bänder gleicher Länge unterteilt ist.

5. Vorrichtung gemäß Patentanspruch 1, wobei die Erstellung des abgeleiteten Signals über eine polynomische Annäherung des Signals mit dem Kriterium der kleinsten Fehlerquadrate erfolgt.

6. Vorrichtun gemäß Patentanspruch 1, wobei man die Globalform der langsamen Phase (f(ti) durch die Minimierung der Summe $F = \Sigma i1 (pi) * (y(ti) - f(ti))^2$ erhält; 1 (pi) ist hierbei die Zugehörigkeitsfunktion eines für das Signal charakteristischen Kurvenpunktes und y (ti) entspricht dem Wert des Signals an einem bestimmten Zeitpunkt (ti).

7. Vorrichtung gemäß Patentanspruch 2, wobei die Globalform der langsamen Phase durch Minimierung der Summe $F = \Sigma i1(pi) * (y(ti) - f(ti)2$, f(ti) bestimmt wird; es handelt sich hierbei um eine polynomische Funktion.

8. Vorrichtung gemäß einem der sieben Patentansprüche 1 bis 7, wobei diese für die Verarbeitung eines nystagmatischen Signals bestimmt ist.

9. Vorrichtung gemäß einem der siehen Patentansprüche 1 bis 7, wobei diese für die automatische Unterteilung eines elektrischen Signals in langsame und schnelle Phasen bestimmt ist.

10. Vorrichtung gemäß einem der neum Patentansprüche 1 bis 9, wobei diese mindestens über einem Speicher für das digitale Signal, für das Differential des besagten digitalen Signals und für das aus den langsamen Phasen kumulierte Signal sowie über einen arithmetischen Prozessor verfügt.

FIG. 1

Position
Table

5

2   2   3
Position
Œil

3        3

1

Position
Cumulée
de la
Phase
Lente

4

FIG. 2

9

1
2
3
4
5
6
7
8
9
10
11
12
13
14
n

6

7

8

FIG. 3

$m_1(n)$

$m_2(n)$

$m_3(n)$

$m_1(n).m_2(n).m_3(n)$

10  11  12  13

$n$

FIG. 4

$n$

FiG.5

$m_1(n)$

$m_2(n)$

$m_3(n)$

$m_1(n).m_2(n).m_3(n)$

19

20

21

22

$n$

FiG. 6

1

2

3

4

5

6

7

$n$

18

15

16

17

# FIG.7

$m_1(n)$

$\underset{23}{}$

$m_2(n)$

$\underset{24}{}$

$m_3(n)$

$\underset{25}{}$

$m_1(n).m_2(n).m_3(n)$

$\underset{26}{}$